# EUROPEAN PATENT SPECIFICATION

(11) **EP 2 378 953 B1**
(45) Date of publication and mention of the grant of the patent: **16.08.2017**
(21) Application number: 09774748.9
(22) Date of filing: 02.12.2009
(51) Int. Cl.: A61B 5/00

(54) **DEVICE FOR OPTICAL DETECTION OF THE CONDITION OF JOINTS**
VORRICHTUNG ZUM OPTISCHEN NACHWEIS DES ZUSTANDS VON GELENKEN
DISPOSITIF DE DÉTECTION OPTIQUE DE L'ÉTAT D'ARTICULATIONS

(30) Priority: 05.12.2008 EP 08170851
(43) Date of publication of application: 26.10.2011
(73) Proprietor: Hemics B.V., 5617 BC Eindhoven (NL)
(72) Inventor: RENSEN, Wouter, H., J., 5656 AE Eindhoven (NL)
(74) Representative: Nederlandsch Octrooibureau
(86) International application number: PCT/IB2009/055463
(87) International publication number: WO 2010/064202

(56) References cited:
- EP-A1- 1 566 142
- EP-A2- 1 745 739
- WO-A2-2007/018921
- WO-A2-2009/147560
- US-A- 5 423 322
- US-B1- 7 324 848
- HIELSCHER, ANDREAS H.; LASKER, JOSEPH M.; FOUG, CHRIS J.; DWYER, EDWARD;: "Imaging of Hemodynamic Effects in Arthritic Joints with Dynamic Optical Tomography" PROC. SPIE, vol. 6629, 6629_22, 12 July 2007 (2007-07-12), pages 66290N-1-66290N-10, XP040242957 DOI: 10.1117/12.728576
- LASKER JOSEPH ET AL: "Digital-signal-processor-based dynamic imaging system for optical tomography" REVIEW OF SCIENTIFIC INSTRUMENTS, AIP, MELVILLE, NY, US LNKD- DOI:10.1063/1.2769577, vol. 78, no. 8, 31 August 2007 (2007-08-31), pages 83706-83706, XP012104149 ISSN: 0034-6748
- GIBSON A P ET AL: "TOPICAL REVIEW; Recent advances in diffuse optical imaging; Topical review" PHYSICS IN MEDICINE AND BIOLOGY, TAYLOR AND FRANCIS LTD. LONDON, GB LNKD- DOI:10.1088/0031-9155/50/4/R01, vol. 50, no. 4, 21 February 2005 (2005-02-21), pages R1-R43, XP020084510 ISSN: 0031-9155

## Description

### FIELD OF INVENTION

The present invention relates to an optical detection method and to a device for optical detection of the condition of joints.

### BACKGROUND OF THE INVENTION

In the context of the present application, the term light is to be understood to mean non-ionizing electromagnetic radiation, in particular with wavelengths in the range between 400 nm and 1400 nm. The term body part means a part of a human or animal body. The term blocking covers both complete blocking and blocking to a substantial extent.

In general, the present invention relates to optical detection of joint conditions, in particular to the optical detection of joint diseases such as rheumatoid arthritis (RA). The treatment of such joint diseases is staged. Usually, a patient first receives pain killers. These are frequently followed by non-steroid anti-inflammatory drugs (NSAIDs) and disease modifying anti-rheumatic drugs (DMARDs). In many cases, the last stage in treatment with drugs is the use of biological therapies. In particular the last category is expensive and treatment can cost tens of thousands of dollars per year per patient. Additionally, the drugs used in later stages of treatment often cause more severe side effects. With respect to such joint diseases, medical professionals base their decisions on changes in therapy on disease activity which is given by the number and the severity of inflamed joints.

Since rheumatoid arthritis is a progressive disease and early diagnosis and start of treatment can help postponing adverse effects and high costs of treatment, there is a demand for methods and devices for providing satisfactory information about the condition of joints and which assist a medical professional to come to a conclusion with respect to the actual joint condition.

It has been found in time-dependent measurements using non-targeted fluorescent dyes administered to the patient that perfusion dynamics in diseased joints are different as compared to normal healthy joints. However, in the clinical practice of rheumatologists, administration of contrast agents is impractical in most cases.

As an alternative, it has been proposed to use Diffuse Optical Tomography (DOT) to image joints for providing information about their condition. In a research project, venous blood flow to a body part has been temporarily obstructed by means of a pressure cuff and a single joint has been imaged by means of DOT. In such studies, it has been found that optical parameters exist which correlate with the presence of rheumatoid arthritis (RA).

For example, it is known that inflammation can be recognized by a change in perfusion. Blood constituents, in particular both oxygenated and deoxygenated hemoglobin have distinct optical characteristics compared to other constituents of the human or animal body and thus can in principle be optically detected.

A device and method for detecting the condition of a joint is described in WO 2009/147560 A2. The document describes measuring the inflammation of a joint by analyzing changes in spectral transmission of joints and other parts of, for instance, a patient's hand before, during, and after (partial) occlusion of blood flow in the patient's arm and hand. Blood flow is occluded using a pressure cuff applied around the patient's arm. Application of a pressure cuff can take time and requires some experience and, consequently, the help of an extra person (e.g. a nurse). Having an inflated cuff around the patient's arm for an extended period of time and can be uncomfortable for the patient and carries the, very small, risk of health damage for the patient by creating a blood clot. Moreover, the cuff and its accompanying equipment, a pump and control electronics, increase the costs of the device and involve mechanical parts that have a relatively high risk of breaking down.

The publication by HIELSCHER, Andreas H. et al: "Imaging of Hemodynamic Effects in Arthritic Joints with Dynamic Optical Tomography", Proc. SPIE, vol. 6629, 6629_22, 12 July 2007 (2007-07-12), pages 66290N-1-66290N-10, XP040242957, DOI: 10.1117/12.728576, which also discloses the application of an occlusion cuff, discloses the features of the preamble of claim 1.

### SUMMARY OF THE INVENTION

It is an object of the invention to provide a device and method for detecting the condition of a joint which provide information about the condition of a joint without the need for a pressure cuff.

The object is solved by a device according to claim 1. The invention is based on the recognition that a patient's heartbeat results in pressure pulses through the patient's of vascular system. Instead of a pressure pulse induced in a patient's vascular system by blocking blood flow using a pressure cuff, a pressure pulse induced by the patient's own heartbeat can also be used to determine the reaction of a body part to such a pressure pulse. However, if detection is to be based on heartbeat-in use pressure pulses, the sampling rate during detection should be higher than the frequency of the patient's heartbeat to provide proper sampling of heartbeat-modulated optical signals detected from the patient. Since the attenuation of the light used for irradiation is locally detected for two distinct positions of which at least one is a joint, differences in the optical properties of the at least one joint compared to the at least one other portion of the body part can be detected. The response of the at least one joint to changes in blood flow compared to the at least one other portion can be detected. During inflammation of a joint, the number and properties of blood vessels (capillaries) in the joint change. This effect together with the specific light absorption of blood is used for measuring the condition of a joint. Due to the measurements under different blood flow conditions (different blood flow conditions being induced during the different phases of a blood pressure pulse induced by the patient's heartbeat), the signal resulting from blood can be separated from signals resulting from other sources of light attenuation in the body. Since at least one joint and at least one other body portion of the body part (e.g. next to the joint) are measured, joint-specific results are achieved and contributions from tissues which are present in both the joint and the other body portion (such as fat, skin, etc.) can be separated. As a result, a signal which is joint-specific for changes in blood content can be obtained. Separate measurements to identify the composition (e.g. bone, fat, skin, etc.) of the body part can be omitted. As a consequence, valuable information about the joint condition and/or disease activity is provided to a medical professional.

More generally, instead of a subject's heartbeat frequency use might be made of a reference frequency of which the subject's heartbeat frequency is only one example. Another reference frequency might be the subject's breathing frequency. The breathing frequency is coupled to the heartbeat frequency. Consequently, the breathing frequency might be used as a reference frequency. The value of the reference frequency to be used depends on how the reference frequency is coupled to the heartbeat frequency.

An embodiment of the device according to the invention is characterized in that the sampling frequency is at least twice the frequency of the subject's heartbeat. This embodiment has the advantage that during sampling no information is lost as this embodiment satisfies the Nyquist sampling theorem.

A further embodiment of the device according to the invention is characterized in that the sampling frequency is at least 100 Hz, preferably at least 120 Hz, preferably at least 150 Hz, preferably at least 200 Hz, preferably at least 250 Hz, preferably at least 300 Hz, preferably at least 400 Hz. This embodiment has the advantage that, depending on a subject's heartbeat frequency, the Nyquist sampling theorem is satisfied. When a subject is relaxed and resting, sampling frequencies of at least 100 Hz, 120 Hz, and 150 Hz will usually be sufficient to satisfy the sampling theorem. However, when a subject is exercising or his heartbeat frequency is elevated for any other reason, sampling frequencies of at least 200 Hz, 250 Hz, and 300 Hz may be required to satisfy the sampling theorem. A sampling frequency of at least 400 Hz will satisfy the sampling theorem regardless of the subject's actual heartbeat frequency. Consequently, no information about the subject's actual heartbeat frequency is then required to ensure proper sampling.

A further embodiment of the device according to the invention is characterized in that the device further comprises a heartbeat sensor for detecting the frequency of the subject's heartbeat, the heartbeat sensor being coupled to the measurement unit such that the sampling frequency is determined based on the frequency of the subject's heartbeat detected by the heartbeat sensor. This embodiment has the advantage that it offers an easy way to couple a subject's actual heartbeat frequency to the sampling frequency with which local attenuation data is obtained.

A further embodiment of the device according to the invention is characterized in that the measurement unit comprises a light source unit capable of emitting light of at least two distinct wavelengths. This embodiment has the advantage that a first wavelength can be chosen such that blood has a higher absorption for light of this first wavelength and a second wavelength can be chosen such that the absorption of blood is lower or comparable to surrounding tissue for a light of this second wavelength. Thus, more detailed information about perfusion of the at least one joint and the at least one other portion of the body part are provided and can be analyzed for judging the condition of the at least one joint.

A further embodiment of the device according to the invention is characterized in that the device further comprises an exercise device for having the subject exercise to affect the subject's heartbeat and/or breathing. This embodiment has the advantage that valuable data for judging the condition of a joint can be obtained by obtaining local attenuation data at different heartbeat frequencies and/or under different breathing conditions. Having a subject exercise is an easy means to change the subject's heartbeat frequency and/or change the subject's breathing. Having the subject exercise also results in a change of the oxygen content of the subject's blood. This change in oxygen content will result in a change in the attenuation of light by blood in a body part of the subject, for instance, a joint. Detecting this change can offer valuable information for judging the condition of a joint.

A further embodiment of the device according to the invention is characterized in that the exercise device is a bike ergometer. This embodiment has the advantage that a bike ergometer is a particularly suitable platform on which to combine detection of the local attenuation of light with subjecting a subject to exercises. While doing the exercises, the subject can sit comfortably on the bike with his hands on the steer. At least part of the measurement unit can be integrated into the steer.

The object of the invention is further solved by an optical detection method according to claim 9. The method achieves the advantages described above with respect to the device.

An embodiment of the method according to the invention is characterized in that the at least one other portion of the body part is another joint. This embodiment has the advantage that the response of different joints to changes in blood flow can be compared and information about differences in the condition of several joints is provided. In a preferred embodiment, all joints in both hands of a patient are measured simultaneously.

A further embodiment of the method according to the invention is characterized in that the results of distinct local attenuation measurements for the at least one joint and for the at least one other portion of the body part which are acquired substantially simultaneously are compared to each other. This embodiment has the advantage that valuable information for judging the condition of the at least one joint can be obtained by comparing data obtained for the joint with data obtained substantially simultaneously from the at least one other portion of the body part. Comparing data that has been obtained substantially simultaneously has the advantage that changes affecting the at least one joint and the at least one other portion simultaneously are cancelled, leaving only information relating to time-independent differences between the at least one joint and the at least one other portion.

A further embodiment of the method according to the invention is characterized in that during acquisition of the distinct local attenuation measurements, the body part is immersed in an optical matching medium. This embodiment has the advantage that optical boundary effects and the dynamic range of intensities to which a detector is subjected are reduced.

A further embodiment of the method according to the invention is characterized in that the method further comprises the additional step of:
having the subject exercise to affect the subject's heartbeat and/or breathing.

This embodiment has the advantage that valuable data for judging the condition of a joint can be obtained by obtaining local attenuation data at different heartbeat frequencies and/or under different breathing conditions. This was already discussed in relation to an embodiment of the device according to the invention.

### BRIEF DESCRIPTION OF THE DRAWINGS

Further features and advantages of the present invention will arise from the detailed description of embodiments with reference to the enclosed drawings.
Fig. 1 schematically shows a set-up for optical detection of the condition of joints according to an embodiment.
Fig. 2 schematically shows details of a measurement unit according to an embodiment.
Fig. 3 schematically shows a human hand as an example for a body part with the positions of joints indicated.
Fig. 4 schematically illustrates the results of simultaneous distinct local attenuation measurements for two joints and one other portion of the body part which is not a joint.

### DETAILED DESCRIPTION OF EMBODIMENTS

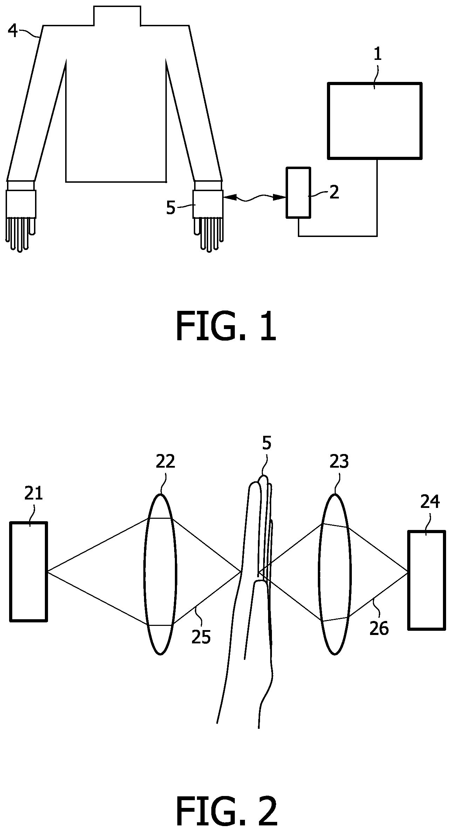

An embodiment of the present invention will now be described with reference to the figures. Fig. 1 schematically shows a set-up for the optical detection of the condition of joints. In the illustration, a human body 4 is schematically shown as a body and a hand forms the body part 5 to be examined. However, it should be noted that the invention is not restricted to human bodies and e.g. animal bodies may be subjected to examination. Further, the body part 5 is not restricted to a hand but may also be formed by another body part comprising at least one joint 6 such as arms, legs, feet, etc.

In the embodiment shown, the device for optical detection of the condition of joints comprises a measurement unit 2 and a control unit 1. The control unit 1 is provided to control the operation of the device and data acquisition. According to the invention data acquisition is arranged such that the sampling frequency with which local attenuation data are obtained is higher than the frequency of the patient's heartbeat. In this way, proper sampling of heartbeat-modulated attenuation signals from the patient is ensured where the same time no blocking of the blood flow by a pressure cuff is needed. The measurement unit 2 is provided to irradiate portions of the body part 5 under examination with light and measure the local attenuation of the light at different positions of the body part 5. For example, in the embodiment shown the measurement unit 2 is formed by a measurement head which will be described in more detail below. The construction of the measurement unit 2 according to the embodiment will be described in further detail with reference to Fig. 2. The device may comprise a heartbeat sensor (not shown) to detect a subject's heartbeat frequency in order to determine a suitable sampling frequency. The device according to the invention may be comprised in a system further comprising an ergometer (not shown). An example of a suitable ergometer is a bike ergometer. An ergometer provides an easy means to have a subject whose body part is being investigated exercise to affect the subject's heartbeat frequency and/or breathing. The system enables measurements under different conditions regarding heartbeat frequency and/or breathing, resulting in valuable information that can aid in determining the condition of a subject's joint. At least part of the device, for instance, a light source unit and/or a detection element (see fig. 2) may be integrated into the ergometer, for instance, into the steer of a bike ergometer. A human subject can then exercise on the bike ergometer holding his hands on the steer while at the same time a joint is measured.

The measurement unit 2 schematically shown in Fig. 2 is adapted for attenuation measurements in transmission geometry. The measurement unit 2 comprises a light source unit 21 emitting a beam of light for irradiating the body part 5. The light source unit 21 comprises at least one light source and appropriate light guides to direct the beam of light to the body part 5. The light source may be formed by a lamp or by one or more lasers and the light guides may for instance be formed by optical fibers. The light source unit 21 is adapted to be capable to emit light of at least two different wavelengths, preferably in the red to near infrared, wherein one wavelength is chosen such that blood has a high absorption and another wavelength is chosen such that the absorption of blood is low or comparable to surrounding tissue. Suitable wavelengths are for instance 600 nm and 805 nm but other wavelengths fulfilling these criteria are possible as well. Wavelengths in the wavelength range between 550 and 980 nm are particularly suitable. Further, an optical component 22 which e.g. may be formed by a lens is provided for directing the light to the body part 5. The optical component 22 is capable of concentrating the light (irradiation light 25) on a specific area of interest (or several specific areas of interest; i.e. specific positions) of the body part 5 as will be described below. A second optical element 23 is provided to collect light emerging from the specific area (or areas) of interest and direct the collected light 26 to a detection element 24. The detection element 24 may for instance be formed by a photodiode, a CCD, an optical guide such as a fiber connecting to a photodiode, or another light detection scheme known in the art.

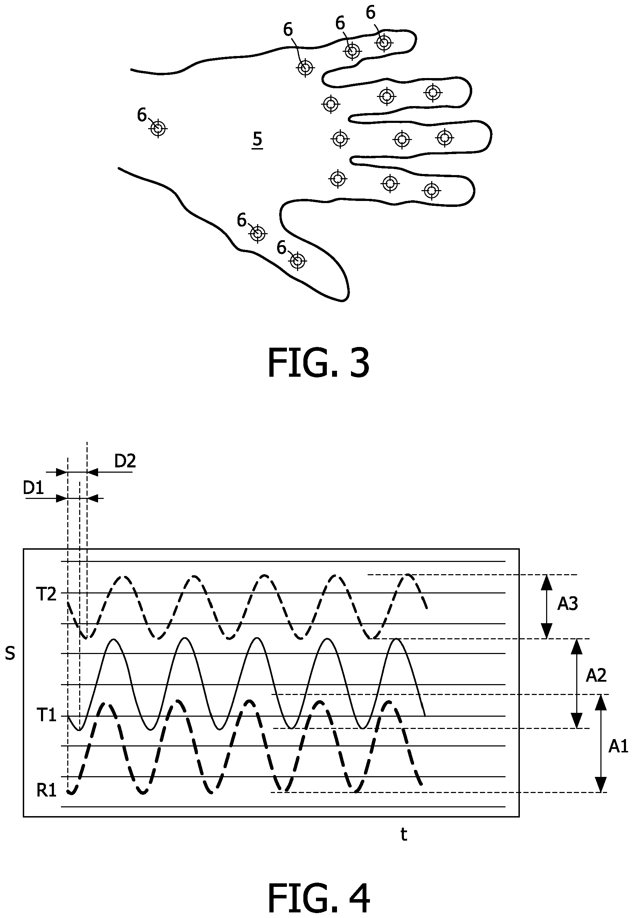

The measurement unit 2 is adapted such that distinct local attenuation measurements for at least two different portions of the body part 5 can be performed. Preferably, multiple joints are measured simultaneously and the time dependent behavior of these multiple joints with respect to each other is analyzed. Still more preferably, all joints of a body part 5 are measured simultaneously. Fig. 3 shows a hand as an example for a body part 5 to be examined and the positions of joints 6 are indicated by crosshairs (it should be noted that not all joints are provided with reference signs). The indicated positions can be used as positions for the local attenuation measurements and additionally positions between these indicated positions can be used for reference attenuation measurements.

In the embodiment shown in Fig. 1, via the measurement unit 2, the control unit 1 detects the spectral characteristics of the body part 5 containing joints 6. The measurement unit 2 now detects spectral changes related to changes in blood flow. Preferably, the perfusion is also compared between joints and other areas of the body part 5. Inflamed joints will have a different perfusion and oxygenation as compared to healthy joints. As a result, the dynamic spectral behavior which is measured by the measurement unit 2 will be different.

Fig. 4 shows an example for the results of attenuation measurements (in transmission geometry) performed simultaneously as a function of time. The trace marked with T1 corresponds to local attenuation measurements at a first joint, the trace marked with T2 corresponds to local attenuation measurements at a second joint, and the trace marked with R1 corresponds to local attenuation measurements at a reference position which is not a joint. The signal changes periodically with the frequency of the patient's heartbeat. The characteristics A1, A2, A3 of the drops occurring in the traces can be different. Inflamed joints can show signs of high perfusion such as an increased drop in transmission compared to other joints or compared to a reference position. The drops reflect differences in modulation depth between the different locations at which measurements are performed. The modulation depth is affected by the changes in blood flow through diseased tissue as compared to blood flow in healthy tissue. Also the time differences D1, D2 between the changes in transmission between the traces T1, T2, and R1 can be used as marker for inflammation and provide important information.

The time-dependent behavior of individual joints, the behavior of joints with respect to each other and with respect to other parts (that can act as a reference) is analyzed.

In the embodiment described above, the measurement unit 2 has been adapted for measurements in transmission geometry, i.e. the body part is irradiated from one side and the light having passed through the body part is measured on the opposite side. In a modification of the embodiment, the measurement unit 2 can be adapted for attenuation measurements in reflection geometry. In this case, irradiation and detection are performed from the same side of the body part 5. In reflection geometry, the optical components 22 and 23 can be combined. It is advantageous to separate the diffuse reflected light from the illumination light. This can be achieved e.g. by orthogonal polarized spectral imaging (OPSI) or darkfield imaging or other suitable techniques known in the art.

It should be noted that, in the embodiments, the blood flow need not be completely blocked but a substantial reduction of the blood flow may suffice.

A plurality of different ways for implementing the measurement unit 2 exists. It is an essential feature that the local collection of light from multiple portions of the body part 5 under examination is measured. This can e.g. be achieved by illuminating a single spot at a time and detecting a corresponding single spot on the body part 5 and scanning the position of the illumination and detection spot over the body part 5.

A further, more preferred possibility is to illuminate the whole body part 5 and to image the transmitted (or reflected) light with a CCD camera or another suitable camera. However, due to the diffuse transmission, in this case the resolution of the image is limited and light traveling e.g. between fingers may overload the detector.

A still more preferred possibility is to illuminate a discrete number of spots on the body part 5. This implementation has the advantage that less stray light reaches the detector which leads to a higher resolution and that the intensity of all the spots can be adjusted such that only a limited dynamic range is required for the detector.

It is also possible to immerse the body part 5 under examination in an optically matching medium, e.g. a fluid, in order to reduce optical boundary effects and the dynamic range of intensities falling on the detector. In such a technique, a fluid having optical properties (such as the optical absorption coefficient and the reduced scattering coefficient) similar to those of tissue is employed.

Further, to detect different wavelengths, it is possible to alternate the illumination wavelength. It is also possible to illuminate with all required wavelengths simultaneously and separate the different wavelengths in the detection path, e.g. using filters or a spectrograph.

In a preferred implementation, multiple body parts (e.g. both hands) are measured simultaneously.

Although it has been described with respect to the embodiment that at least two wavelengths are used for illumination, the invention is not restricted to that. For example, a larger number of discrete wavelengths can be used or even a complete spectrum over a certain range of wavelengths (e.g. 650 to 1000 nm). However, acquiring a complete spectrum requires more costly components as compared to a few distinct wavelengths. If several types of tissue components (such as fat, water, etc.) shall be discriminated, it might be advantageous to use more than two distinct wavelengths. Using more wavelengths helps improving the accuracy of the device, however, at increased cost and complexity.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the system claims enumerating several means, several of these means can be embodied by one and the same item of computer readable software or hardware. The mere fact that certain measures are recited in mutually different dependent claims does not indicate that a combination of these measures cannot be used to advantage.

## Claims

1. Device for optical detection of the condition of a joint, the device comprising:
- a measurement unit (2) for irradiating a subject's body part (5) comprising at least one joint (6) with light and locally detecting attenuation of the light at the at least one joint and at least one other portion of the body part (5), wherein the sampling frequency for locally detecting the attenuation of the light is higher than the frequency of the subject's heartbeat, wherein the device is further adapted for measuring the condition of the at least one joint by comparing
a) the response in the detected attenuation of the light at the at least one joint to changes in blood flow to
b) the response in the detected attenuation of the light at the at least one other portion of the body part to said changes in blood flow,
**characterised in that**
said changes in blood flow are changes in blood flow induced during the different phases of a blood pressure pulse induced by the subject's heartbeat.

2. Device according to claim 1, wherein the sampling frequency is at least twice the frequency of the subject's heartbeat.

3. Device according to any one of claims 1-2, wherein the sampling frequency is at least 100 Hz, preferably at least 120 Hz, preferably at least 150 Hz, preferably at least 200 Hz, preferably at least 250 Hz, preferably at least 300 Hz, preferably at least 400 Hz.

4. Device according to any one of claims 1-3, wherein the device further comprises:
- a heartbeat sensor for detecting the frequency of the subject's heartbeat, the heartbeat sensor being coupled to the measurement unit (2) such that the sampling frequency is determined based on the frequency of the subject's heartbeat detected by the heartbeat sensor.

5. Device according to any one of claims 1-4, wherein the measurement unit (2) comprises a light source unit (21) capable of emitting light of at least two distinct wavelengths.

6. Device according to any one of claims 1-5, wherein the device further comprises:
- an exercise device for having the subject exercise to affect the subject's heartbeat and/or breathing.

7. Device according to claim 6, wherein the exercise device is a bike ergometer.

## Patentansprüche

1. Vorrichtung zum optischen Detektieren des Zustands eines Gelenks, wobei die Vorrichtung aufweist:
eine Messeinheit (2) zum Bestrahlen eines Körperteils (5) eines Probanden, das wenigstens ein Gelenk (6) aufweist, mit Licht und zum lokalen Detektieren der Abschwächung des Lichts an dem wenigstens einen Gelenk und an wenigstens einem anderen Körperbereich des Körperteils (5), wobei die Abtastfrequenz zum lokalen Detektieren der Abschwächung des Lichts größer als die Frequenz des Herzschlags des Probanden ist, wobei die Vorrichtung weiter dazu eingerichtet ist, den Zustand des wenigstens einen Gelenks zu messen durch Vergleich von
a) der Reaktion in der detektierten Abschwächung des Lichts an dem wenigstens einen Gelenk auf Veränderungen im Blutfluss mit
b) der Reaktion in der detektierten Abschwächung des Lichts an dem wenigstens einen anderen Bereich des Körperteils auf Änderungen im Blutfluss,
**dadurch gekennzeichnet, dass**
die Änderungen im Blutfluss Änderungen im Blutfluss sind, die während der verschiedenen Phasen des Blutdruckpulses hervorgerufen werden, der durch den Herzschlag des Probanden erzeugt wird.

2. Vorrichtung nach Anspruch 1, wobei die Abtastfrequenz wenigstens doppelt so hoch wie die Frequenz des Herzschlags des Probanden ist.

3. Vorrichtung nach einem der Ansprüche 1 - 2, wobei die Abtastfrequenz wenigstens 100 Hz beträgt, vorzugsweise wenigstens 120 Hz, vorzugsweise wenigstens 150 Hz, vorzugsweise wenigstens 200 Hz, vorzugsweise wenigstens 250 Hz, vorzugsweise wenigstens 300 Hz, vorzugsweise wenigstens 400 Hz beträgt.

4. Vorrichtung nach einem der Ansprüche 1 - 3, wobei die Vorrichtung weiter aufweist:
einen Herzschlagsensor zum Detektieren der Frequenz des Herzschlags des Probanden, wobei der Herzschlagsensor mit der Messeinheit (2) verbunden ist, so dass die Abtastfrequenz auf Grundlage der Frequenz des Herzschlags des Probanden, die von dem Herzschlagsensor detektiert ist, bestimmt wird.

5. Vorrichtung nach einem der Ansprüche 1 - 4, wobei die Messeinheit (2) eine Lichtquelleneinheit (21) aufweist, die zum Ausstrahlen von Licht bei wenigstens zwei verschiedenen Wellenlängen in der Lage ist.

6. Vorrichtung nach einem der Ansprüche 1 - 5, wobei die Vorrichtung weiter aufweist:
eine Trainingsvorrichtung, um den Probanden trainieren zu lassen, um den Herzschlag und/oder die Atmung des Probanden zu beeinflussen.

7. Vorrichtung nach Anspruch 6, wobei die Trainingsvorrichtung ein Fahrradergometer ist.

## Revendications

1. Dispositif pour la détection optique de l'état d'une articulation, le dispositif comprenant :
- une unité de mesure (2) pour irradier une partie de corps (5) d'un sujet comprenant au moins une articulation (6) avec de la lumière et détecter localement une atténuation de la lumière au niveau de la au moins une articulation et d'au moins une autre portion de la partie de corps (5), dans lequel la fréquence d'échantillonnage pour détecter localement l'atténuation de la lumière est supérieure à la fréquence du rythme cardiaque du sujet, dans lequel le dispositif est en outre adapté pour mesurer l'état de la au moins une articulation en comparant
a) la réponse dans l'atténuation détectée de la lumière au niveau de la au moins une articulation à des changements d'écoulement sanguin,
b) la réponse dans l'atténuation détectée de la lumière au niveau de la au moins une autre partie de la partie de corps auxdits changements d'écoulement sanguin,
**caractérisé en ce que**
lesdits changements d'écoulement sanguin sont des changements d'écoulements sanguin induits pendant les différentes phases d'une impulsion de pression sanguine induite par le rythme cardiaque du sujet.

2. Dispositif selon la revendication 1, dans lequel la fréquence d'échantillonnage est au moins deux fois la fréquence du rythme cardiaque du sujet.

3. Dispositif selon l'une quelconque des revendications 1 et 2, dans lequel la fréquence d'échantillonnage est d'au moins 100 Hz, de préférence d'au moins 120 Hz, de préférence d'au moins 150 Hz, de préférence d'au moins 200 Hz, de préférence d'au moins 250 Hz, de préférence d'au moins 300 Hz, de préférence d'au moins 400 Hz.

4. Dispositif selon l'une quelconque des revendications 1 à 3, dans lequel le dispositif comprend en outre :
- un capteur de rythme cardiaque pour détecter la fréquence du rythme cardiaque du sujet, le capteur de rythme cardiaque étant couplé à l'unité de mesure (2) de telle sorte que la fréquence d'échantillonnage est déterminée sur la base de la fréquence du rythme cardiaque du sujet détecté par le capteur de rythme cardiaque.

5. Dispositif selon l'une quelconque des revendications 1 à 4, dans lequel l'unité de mesure (2) comprend une unité de source de lumière (21) capable d'émettre de la lumière d'au moins deux longueurs d'onde distinctes.

6. Dispositif selon l'une quelconque des revendications 1 à 5, dans lequel le dispositif comprend en outre :
- un dispositif d'exercice pour savoir comment un exercice effectué par le sujet va affecter le rythme cardiaque et/ou la respiration du sujet.

7. Dispositif selon la revendication 6, dans lequel le dispositif d'exercice est une bicyclette ergométrique.
